(19) Europäisches Patentamt
European Patent Office
Office européen des brevets

(11) Publication number: **0 149 291**
**B1**

(12) **EUROPEAN PATENT SPECIFICATION**

(45) Date of publication of patent specification: **18.05.88**

(51) Int. Cl.$^4$: **C 07 D 213/61**

(21) Application number: **84300241.1**

(22) Date of filing: **16.01.84**

(54) **Process for the preparation of substituted pyridines.**

(43) Date of publication of application:
**24.07.85 Bulletin 85/30**

(45) Publication of the grant of the patent:
**18.05.88 Bulletin 88/20**

(84) Designated Contracting States:
**BE CH DE FR GB IT LI NL**

(56) References cited:
**EP-A-0 012 117**
**EP-A-0 046 735**
**US-A-4 435 573**

(73) Proprietor: **THE DOW CHEMICAL COMPANY**
**2030 Dow Center Abbott Road P.O. Box 1967**
**Midland, MI 48640 (US)**

(72) Inventor: **Lysenko, Zenon**
**214 West Meadowbrook**
**Midland Michigan (US)**
Inventor: **Pews, Richard Garth**
**4403 Andre**
**Midland Michigan (US)**

(74) Representative: **Allard, Susan Joyce et al**
**BOULT, WADE & TENNANT 27 Furnival Street**
**London EC4A 1PQ (GB)**

**EP 0 149 291 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the preparation of substituted pyridines by a base and transition metal catalyzed ring closure of substituted 5-oxo-pentane-1-nitriles.

EP—A—0046735 teaches the preparation of chloropyridines substituted by methyl, trichloromethyl or trifluoromethyl groups by adding the appropriate aldehyde to acrylonitrile, methacylonitrile or α-trifluoromethacrylonitrile in the presence of a catalyst and cyclizing the resulting intermediate in the presence of hydrogen chloride or a substance which forms hydrogen chloride under the reaction conditions.

EP—A—0012117 discloses a process for the preparation of 2,3,5-trichloropyridine in which trichloroacetaldehyde is reacted in the presence of a catalyst with acrylonitrile and the 2,4,4-trichloro-4-formylbutylnitrile so formed is cyclized to give 2,3,5-trichloropyridine. The catalyst is preferably a compound of a group VIII, VIa, VIIa, Ib or IIb metal.

We have now developed a process for the preparation of substituted pyridines in which the cyclization of a substituted oxopentane nitrile is carried out in the presence of a catalyst comprising a transition metal compound and an organic amine.

Accordingly, the present invention provides a process for preparing compounds having the formula

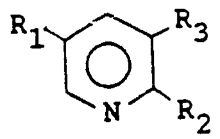

wherein $R_1$ is a $C_{1-4}$ alkyl group, a phenyl group or a hydrogen or halogen atom, $R_2$ is a chlorine or bromine atom and $R_3$ is a fluorine, chlorine or bromine atom which comprises cyclizing a compound having the formula

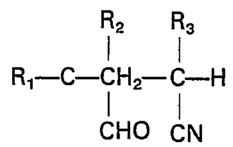

wherein $R_1$, $R_2$ and $R_3$ are as above-defined, in the presence of a catalytic amount of an organic amine base and a transition metal catalyst, the reaction being carried out at a temperature in the range of from 25°C to 250°C in the presence of an inert polar solvent.

The compounds prepared by the process of this invention are advantageously employed as intermediates in the preparation of compounds having insecticidal and/or herbicidal activitiy as described for example, in European Patent Application 0046735.

The reaction may be carried out at any temperature of from 25°C to 250°C, but is preferably carried out at temperatures of from 70°C to 250°C and most preferably at temperatures of 200°C to 220°C

Examples of the catalytic amine are ethylamine, methylamine and cyclohexylamine although isopropylamine is the preferred catalytic amine.

The transition metal catalyst may be, for example, $Ni(C1_2)$ or $CoC1_2(O_3P)_2$. The preferred catalyst is nickel dichlorobis(triphenylphosphine). The reaction is carried out in a suitable inert polar solvent, preferably a nitrile solvent and most preferably acetonitrile.

The invention is further illustrated by the following examples.

Example 1 (comparative)

Preparation of 2,3-dichloro-5-methylpyridine

A solution of 32.343 grams of 2,4-dichloro-4-methyl-5-oxopentanenitrile in 150 ml of acetonitrile and containing 300 mg of nickel dichlorobis(triphenylphosphine) was pumped through a nickel coil having a volume of about 30 ml at the rate of 2.0—2.5 ml/minute. The solution had been preheated to 200°—210°C at a pressure of 250 psig (1825 kilopascals).

Gas chromatographic (G.C.) analysis showed 90% conversion of aldehyde with a 77% yield of 2,3-dichloro-5-methylpyridine.

Example 2

The above experiment was repeated except that 30.908 grams of the oxonitrile were used and 3 ml of isopropylamine were included in the solution in addition to the nickel catalyst. Analysis by G.C. showed a conversion of 94.3% and a yield of 23.9 grams (90.1%).

Example 3 (comparative)

The above experiment was repeated except that 31.7 grams of oxonitrile were used and about 1% by weight (3.1 g) of isopropylamine was employed as the sole catalyst. The yield was 80% by G.C.

Example 4

A solution of 32.1 grams of 2,4-dichloro-4-methyl-5-oxopentanenitrile in 250 ml of acetonitrile containing 3 ml of isopropylamine and about 300 mg of nickel dichlorobis(triphenylphosphine) was heated to reflux for 48 hours. The excess acetonitrile was then removed by vacuum distillation and the residue flask-distilled at 85°—90°C at 666.0Pa (5 mm Hg) on a Kugelrohr. The yield was 27.0 grams of 2,3-dichloro-5-methylpyridine (86%) isolated as a white solid.

Example 5

A stock solution containing 30 g of 2,4-dichloro-4-methyl-5-oxopentane nitrile in 150 ml of acetonitrile was prepared and aliquots of 15—20 ml were withdrawn and charged into 50-ml Fisher-Porter bottles. The indicated catalysts (20 µl) were then individually added to separate bottles. The reaction mixtures were then placed in an oil bath preheated to 185°C and allowed to react for the indicated time, after which they were analyzed by gas chromatography. The results were as follows:

| Catalyst | Time | Percent Conversion | Percent Yield |
|---|---|---|---|
| The Invention | | | |
| Isopropyl amine + 2 mg $CoCl_2 \cdot 6H_2O$ + 2 mg Triphenylphosphine | 1 hour | 88 | 89 |
| Comparative t-Butylamine | 45 min. | 71 | 75 |
| Aniline | 45 min. | 84 | 80 |
| Triethylamine | 1 hour | 86 | 74 |

**Claims**

1. A process for preparing compounds having the formula

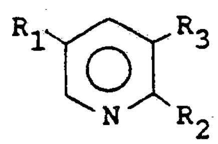

wherein $R_1$ is a $C_{1-4}$ alkyl group, a phenyl group or a hydrogen or halogen atom, $R_2$ is a chlorine or bromine atom and $R_3$ is a fluorine, chlorine or bromine atom, which comprises cyclizing a compound having the formula

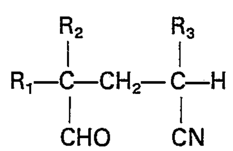

wherein $R_1$, $R_2$ and $R_3$ are as above-defined, in the presence of a catalytic amount of an organic amine base and a transition metal catalyst, the reaction being carried out at a temperature in the range of from 25°C to 250°C in the presence of an inert polar solvent.

2. A process as claimed in claim 1 wherein $R_1$ is a methyl group, and $R_2$ and $R_3$ are both chlorine atoms.

3. A process as claimed in claim 1 wherein $R_1$ is an ethyl group and $R_2$ and $R_3$ are both chlorine atoms.

4. A process as claimed in any one of the preceding claims wherein the organic amine base is methylamine, ethylamine, isopropylamine or cyclohexylamine.

5. A process as claimed in any one of the preceding claims wherein the transition metal catalyst is nickel dichlorobis(triphenylphosphine).

6. A process as claimed in any one of the preceding claims wherein the temperature is in the range of from 70° to 250°C.

**Patentansprüche**

1. Verfahren zur Herstellung von Verbindungen der Formel

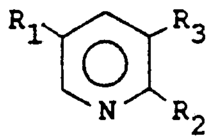

worin $R_1$ eine $C_{1-4}$ Alkylgruppe, eine Phenylgruppe oder ein Wasserstoff- oder Halogenatom ist, $R_2$ ein Chlor- oder Bromatom und $R_3$ ein Fluor-, Chlor- oder Bromatom ist, umfassend zyklisieren einer Verbindung der Formel

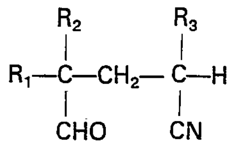

worin $R_1$, $R_3$ und $R_3$ wie oben definiert sind, in Anwesenheit einer katalytischen Menge einer organischen Aminbase und eines Übergangsmetall-Katalysators, wobei die Reaktion bei einer Temperatur im Bereich von 25°C bis 250°C in Anwesenheit eines inerten polaren Lösungsmittels durchgeführt wird.

2. Verfahren nach Anspruch 1, worin $R_1$ eine Merthylgruppe, und $R_2$ und $R_3$ beide Chloratome bedeuten.

3. Verfahren nach Anspruch 1, worin $R_1$ eine Ethylgruppe und $R_2$ und $R_3$ beide Chloratome sind.

4. Verfahren nach einem der vorhergehenden Ansprüche, worin die organische Aminbase ein Methylamin, Ethylamin, Iospropylamin oder Cyclohexylamin ist.

5. Verfahren nach einem der vorhergehenden Ansprüche, worin der Übergangsmetall-Katalysator Nickel-dichlorbis(triphenylphosin) ist.

6. Verfahren nach einem der vorhergehenden Ansprüche, worin die Temperatur in dem Bereich zwischen 70°C und 250°C liegt.

**Revendications**

1. Procédé de préparation de composés répondant à la formule:

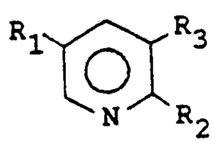

dans laquelle $R_1$ est un groupe alkyle en $C_1$—$C_4$, un groupe phényle ou un atome d'hydrogène ou d'halogène, $R_2$ est un atome de chlore ou de brome et $R_3$ est un atome de fluor, de chlore ou de brome, qui comprend la cyclisation d'un composé répondant à la formule

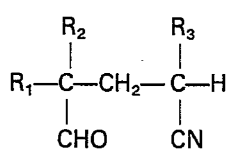

dans laquelle $R_1$, $R_2$ et $R_3$ sont tels que définis ci-dessus, en présence d'une quantité catalytique d'une base de type amine organique et d'un catalyseur à métal de transition, la réaction étant réalisée à une température dans l'intervalle de 25°C à 250°C, en présence d'un solvant polaire inerte.

2. Procédé selon de revendication 1, dans lequel $R_1$ est un groupe méthyle, et $R_2$ et $R_3$ sont tous deux des atomes de chlore.

3. Procédé selon la revendication 1, dans lequel $R_1$ et un groupe éthyle, et $R_2$ et $R_3$ sont tous deux atomes de chlore.

4. Procédé selon l'une quelconque des revendications précédentes, dans lequel la base de type amine organique est la méthylamine, l'éthylamine, l'isopropylamine ou la cyclohexylamine.

5. Procédé selon l'une quelconque des revendications précédentes, dans lequel le catalyseur à métal de transition est le nickel-dichlorobis(triphénylphosphine).

6. Procédé selon l'une quelconque des revendications précédentes, dans lequel la température est dans l'intervalle de 70° à 250°C.